# EUROPEAN PATENT APPLICATION

(11) **EP 4 407 313 A1**
(43) Date of publication of application: **31.07.2024**
(21) Application number: 22872869.7
(22) Date of filing: 20.09.2022
(51) Int. Cl.: G01N 33/48

(54) **BLOOD CELL SEPARATION AGENT, AND BLOOD CELL SEPARATION METHOD USING SAME**

(30) Priority: 21.09.2021 JP 2021152944
(71) Applicant: Nitto Boseki Co., Ltd., Fukushima-shi Fukushima 960-8161 (JP)
(72) Inventor: MASUYA Amiko, Koriyama-shi, Fukushima 963-8061 (JP); WATANABE Koji, Koriyama-shi, Fukushima 963-8061 (JP); TERUUCHI Yuya, Koriyama-shi, Fukushima 963-8061 (JP); TERUUCHI Yoko, Koriyama-shi, Fukushima 963-8061 (JP); TAKEUCHI Minoru, Koriyama-shi, Fukushima 963-8061 (JP)
(74) Representative: Hoffmann Eitle
(86) International application number: PCT/JP2022/034900
(87) International publication number: WO 2023/048115

(57) **Abstract**

Provided is: a blood cell separation agent capable of exhibiting excellent blood cell separation ability with avoiding contamination of a sample with a polymer contained in the agent for use in blood cell separation, and a method of separating blood cell using the same.

A blood cell separation agent comprising: an inorganic carrier; and polymer(s) having structural units with amino groups, wherein at least some of the amino group(s) are a primary, secondary, or tertiary amino group; at least some of the polymer(s) are carried on the inorganic carrier, and others of the polymer(s) are crosslinked to the polymer(s) carried on the inorganic carrier.

## Description

### Technical Field

The present invention relates to an agent for use in blood cell separation, and a method of separating blood cells using the same, and more particularly, it relates to an agent for use in blood cell separation comprising a specific polymer carried on an inorganic carrier, and a method of separating blood cell using the same.

### Background Art

Blood is a specimen used in various biochemical or immunological tests, and is usually separated into plasma or serum and blood cells to be respectively subjected to various analyses. Recently, there is a need to shorten the time between specimen collection and presentation of analysis results, and attempts have been made to shorten the time required for blood cell separation and to perform processes of from the separation to the analysis in sequence in many clinical tests.

Conventionally, a centrifugation method has been commonly employed for separation between a plasma or serum and a blood cell, and simpler and faster blood cell separation methods that do not require centrifugation are under investigation.

As for the simpler and faster blood cell separation method not requiring centrifugation, for example, proposed been have methods of separating blood into serum and blood clots by adding, to collected blood, a solution containing a cholic acid-based surfactant and an acid (Patent Document 1) or a specific quaternary amine sulfone polymer (Patent Document 2). These methods may be, however, affected by contamination of serum or plasma with aggregating agents.

Proposed been also has a simple test device comprising a reaction pad containing a reagent to be reacted with a target soluble component, and a filtering pad for separating soluble components and a blood cell (Patent Document 3), and this filtering pad is composed of a carrier matrix which is impregnated with thrombin and lectin. In this device, blood is introduced to the filtering pad, and plasma or serum separated from blood cells is introduced into the reaction pad for analysis. Therefore, this device has a merit that the separated plasma or serum is directly subjected to analysis. On the other hand, the plasma or serum separated by the filtering pad may be contaminated with thrombin and lectin, but particular consideration is not given to this issue in this device.

Alternatively, a blood filter, wherein a blood coagulant is impregnated in or adhered to a glass nonwoven fabric, has been proposed (Patent Document 4), and poly(diallyl dimethyl ammonium chloride) is used as the blood coagulant. In this blood filter, however, the blood coagulant may be dissociated from the glass nonwoven fabric to contaminate the separated plasma or serum, but particular consideration is not given to this issue in this filter, either.

### Prior Art Documents

### Patent Document

Patent Document 1: JP 2018-059791 A
Patent Document 2: JP 1984-26062 A
Patent Document 3: JP 1992-212060 A
Patent Document 4: JP 2020-085887 A

### Summary of Invention

### Technical Problem

In contrast to the prior art, an object of the present invention is to provide an agent for use in blood cell separation capable of exhibiting excellent blood cell separation ability with avoiding contamination of a sample with a polymer contained in the agent for use in blood cell separation, and a method of separating blood cell using the same.

### Solution to Problem

While evaluating blood cell separation ability by treating blood with blood cell separation agents in which various polymers having blood cell coagulation ability are adhered to inorganic carriers, the present inventors have encountered a phenomenon that polymers contained in blood cell separation agents are desorbed from a carrier, and hence have investigated the cause, resulting in finding that the main cause is pH change. These events can cause contamination of separated samples with the polymers, which affects the analysis system, and the present inventors have therefore made earnest studies to solve this problem. As a result, the present inventors have successfully obtained an agent for use in blood cell separation from which a polymer contained in the agent is not desorbed even through pH change, and thus, the present invention has been accomplished.

Specifically, the present invention relates to:
[1] An agent for use in blood cell separation comprising an inorganic carrier, and polymer(s) having a constituent unit with amino group(s), wherein at least some of the amino group(s) are a primary amino group, a secondary amino group, or a tertiary amino group, at least some of the polymer(s) are carried on the inorganic carrier, and others of the polymer(s) are crosslinked to the polymer(s) carried on the inorganic carrier.

The following [2] to [19] are all preferable aspects or embodiments of the present invention:
[2] The agent for use in blood cell separation according to [1], wherein at least some of the amino group(s) are a secondary amino group, or a tertiary amino group.
[3] The agent for use in blood cell separation according to [1] or [2], wherein the crosslinking is made by a crosslinking agent that is a compound having, in total, at least two functional groups capable of forming a covalent bond with the amino group(s).
[4] The agent for use in blood cell separation according to any one of [1] to [3], wherein the polymer(s) are (a1) an allylamine (co)polymer, (a2) a diallylamine (co)polymer, or (a3) a polyethyleneimine.
[5] The agent for use in blood cell separation according to [4], wherein the polymer(s) are (a2) a diallylamine (co)polymer or (a3) a polyethyleneimine.
[6] The agent for use in blood cell separation according to any one of [1] to [5], wherein the weight average molecular weight of the polymer(s) is in the range of 3,000 to 200,000.
[7] The agent for use in blood cell separation according to any one of [1] to [6], wherein the inorganic carrier is an inorganic particle having a primary particle size between 0.05 and 500 µm.
[8] The agent for use in blood cell separation according to any one of [1] to [7], wherein the polymer(s) is carried on the inorganic carrier by adsorption through electrostatic interaction.
[9] The agent for use in blood cell separation according to any one of [1] to [7], wherein the polymer(s) are carried on the inorganic carrier by a covalent bond.
[10] The agent for use in blood cell separation according to [9], wherein the covalent bond is formed by silane coupling.
[11] A method of separating blood cells, comprising the step of contacting a specimen containing blood cells with the agent for use in blood cell separation according to any one of [1] to [10].
[12] A method for producing an agent for use in blood cell separation, comprising the steps of:
   carrying polymer(s) having a constituent unit with amino group(s) on an inorganic carrier; and
   reacting, before or after carrying the polymer(s) on the inorganic carrier, the polymer(s) with a crosslinking agent to link the polymer(s) with each other by crosslinking,
   wherein at least some of the amino group(s) are a primary amino group, a secondary amino group, or a tertiary amino group.
[13] The method according to [12], wherein at least some of the amino group(s) are a secondary amino group, or a tertiary amino group.
[14] The method according to [12] or [13], wherein the polymer(s) are (a1) an allylamine (co)polymer, (a2) a diallylamine (co)polymer, or (a3) a polyethyleneimine.
[15] The method according to [14], wherein the polymer(s) are (a2) a diallylamine (co)polymer, or (a3) a polyethyleneimine.
[16] The method according to any one of [12] to [15], wherein the polymer(s) are carried on the inorganic carrier by adsorption through electrostatic interaction and/or by a covalent bond.
[17] The method according to [16], wherein the polymer(s) are covalently bonded to the inorganic carrier with a silane coupling agent.
[18] The method according to [16], wherein the polymer(s) are contacted with the inorganic carrier in an alkaline solution to be adsorbed on the inorganic carrier by electrostatic interaction.
[19] The method according to any one of [12] to [18], wherein the crosslinking agent is a compound having, in total, at least two functional groups capable of forming a covalent bond with the amino group(s).

According to an agent for use in blood cell separation of the present invention, the polymer(s) carried on the inorganic carrier are not desorbed, and hence a separated sample is not contaminated with the polymer(s). Therefore, accurate measurements can be conducted in various analysis systems. Even when a sample to be introduced into an analysis system is required to have a pH suitable for the analysis, blood cells can be separated under a pH condition suitable for the analysis, and hence a sample resulting from the separation process can be directly subjected to the analysis.

### Brief Description of Drawings

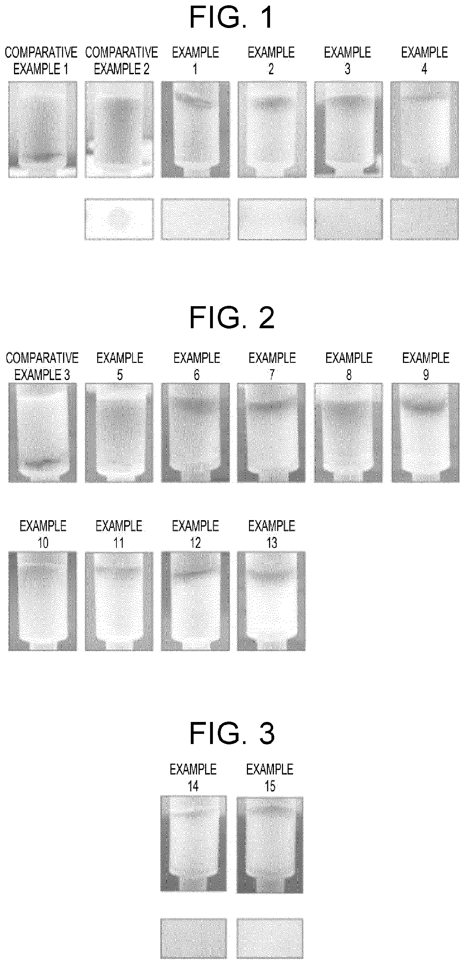

[Fig. 1] Fig. 1 illustrates the results from evaluation of the blood cell separation agents (using silica beads as a carrier) of Examples 1 to 4 and Comparative Examples 1 and 2 for blood cell separation ability and desorption of a polymer from a carrier through pH change. A red portion corresponds to an erythrocyte, and it is understood that blood cells were held in the upper portion of the reservoirs in Examples 1 to 4. It was confirmed in Comparative Example 2 that blood cells moved from the upper portion of the reservoir downward. On the other hand, in Comparative Example 1, blood cells reached the bottom of the reservoir to flow out of the reservoir. In Examples 1 to 4, a substance stained with Ponceau S was not found in a buffer at pH 5.0. On the other hand, in Comparative Example 2, a substance stained with Ponceau S was found in a buffer at pH 5.0, and it is understood that the polymer was desorbed from the carrier.
[Fig. 2] Fig. 2 illustrates the results from evaluation of the blood cell separation agents (using glass beads as a carrier) of Examples 5 to 13 and Comparative Example 3 for blood cell separation ability. A red portion corresponds to blood cells, and it is understood that blood cells were held in the upper portion of the reservoirs in Examples 6, 7, 9, and 11 to 13. On the other hand, in Comparative Example 3, blood cells reached the bottom of the reservoir to flow out of the reservoir. It was confirmed in Examples 5, 8 and 10 that blood cells somewhat moved from the upper portion of the reservoir downward.
[Fig. 3] Fig. 3 illustrates the results from evaluation of blood cell separation agents of Examples 14 and 15 (using silica beads as a carrier) for blood cell separation ability and desorption of a polymer from a carrier through pH change. A red portion corresponds to blood cells, and it is understood that blood cells were held in the upper portion of the reservoirs in Examples 14 and 15. In Examples 14 and 15, a substance stained with Ponceau S was not found in a buffer at pH 5.0, and it is understood that the polymer was not desorbed from the carrier.

### Description of Embodiments

An agent for use in blood cell separation of the present invention is an agent for use in blood cell separation comprising an inorganic carrier, and polymer(s) having a constituent unit with amino group(s), wherein at least some of the amino group(s) are a primary amino group, a secondary amino group, or a tertiary amino group; at least some of the polymer(s) are carried on the inorganic carrier; and the polymer(s) carried on the inorganic carrier are linked to others of the polymer(s) through crosslinking.

Thus, an agent for use in blood cell separation of the present invention contains polymer(s) having a constituent unit with amino group(s), at least some of which are a primary amino group, a secondary amino group, or a tertiary amino group (hereinafter referred to also as "Specific Polymer(s)").

An agent for use in blood cell separation of the present invention, comprises Specific Polymer(s), and can hence realize excellent technical effects such as excellent blood cell separation performance. Specific Polymer(s) are stably carried on an inorganic carrier, which allows blood cells to be captured and separated efficiently and with favorable handleability.

An agent for use in blood cell separation of the present invention may contain only one type of Specific Polymer(s), or may contain a combination of two or more types of Specific Polymer.

### Specific Polymer(s)

Specific Polymer(s) are only required to be a (co)polymer having at least one constituent unit having, in the molecule, amino group(s), at least some of which are a primary amino group, a secondary amino group, or a tertiary amino group (hereinafter referred to also as the "Specific Constituent Unit(s)"), and is not particularly limited otherwise.

Here, the term "(co)polymer" encompasses both a homopolymer and a copolymer, and accordingly, Specific Polymer(s) may be a homopolymer, which consists of Specific Constituent Unit(s), or may be a copolymer, which has another constituent unit in addition to Specific Constituent Unit(s).

Specific Polymers are only required to have at least one Specific Constituent Unit in the molecule, but from the viewpoint of a blood cell separation speed, it preferably has a plurality of Specific Constituent Units. More specifically, the ratio of Specific Constituent Unit(s) to all constituent units of Specific Polymer is preferably 1 mol% or more, more preferably 10 to 100 mol%, and particularly preferably 50 to 100 mol%.

Specific Polymer(s) may have, in the molecule, only one type of Specific Constituent Unit(s), or may have two or more types of Specific Constituent Units. When Specific Polymer(s) has two or more types of Specific Constituent Units, the above-described ratio of Specific Constituent Unit(s) to all constituent units of Specific Polymer is calculated based on the total molar number of the two or more types of Specific Constituent Units.

Specific Constituent Unit(s) are only required to have amino groups, at least some of which are a primary amino group, a secondary amino group, or a tertiary amino group, and is not particularly limited otherwise. The amino group(s) in Specific Constituent Unit(s), at least some of which are a primary amino group, a secondary amino group, or a tertiary amino group, allows Specific Polymers to be more efficiently crosslinked with each other to be more stably carried on an inorganic carrier. From the viewpoint of realizing favorable blood cell separation performance, at least some of the amino group(s) in Specific Constituent Unit(s) are preferably a secondary amino group or a tertiary amino group.

The amino group(s) in Specific Constituent Unit(s) may be a free amino group, or a cationic amino group having an addition salt or the like.

From the viewpoints of availability, structure controllability, solubility in water and the like, as Specific Polymer, (a1) an allylamine (co)polymer, (a2) a diallylamine (co)polymer, and (a3) a polyethyleneimine can be preferably used.

Only one of (a1) an allylamine (co)polymer, (a2) a diallylamine (co)polymer, and (a3) a polyethyleneimine may be used, or two or more of these may be used together.

From the viewpoint of realizing favorable blood cell separation performance, (a2) a diallylamine (co) and/or (a3) a polyethyleneimine are particularly preferably used as Specific Polymer(s).

### (a1) Allylamine (Co)Polymer

(a1) an allylamine (co)polymer, used preferably as Specific Polymer of the present invention, has a constituent unit derived from (A1) a monoallylamine-based monomer. The monoallylamine-based monomer has an amino group, and a constituent unit derived from (A1) the monoallylamine-based monomer hence corresponds to Specific Constituent Unit(s) in (a1) an allylamine (co)polymer.

The amino group(s) in a constituent unit derived from (A1) a monoallylamine-based monomer may be any of primary, secondary, and tertiary, and from the viewpoint of realizing favorable blood cell separation performance, it is preferably a secondary amino group, or a tertiary amino group.

More specifically, a constituent unit derived from (A1) a monoallylamine-based monomer preferably has a structure represented by the following formula (I), or a structure of an addition salt thereof.

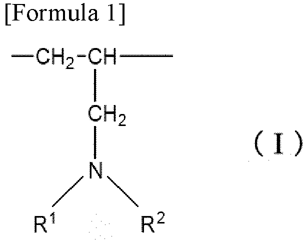

In the formula (I), R¹ and R² each independently represent a hydrogen atom, or a monovalent organic group, and more preferably, R¹ and R² each independently represent a hydrogen atom, an alkyl group having 1 to 12 carbon atoms, or a cycloalkyl group having 5 to 6 carbon atoms.

An alkyl group having 1 to 12 carbon atoms, which is a preferable alternative of R¹ and R², may be either linear or branched, or may be an aralkyl group. Examples thereof include a methyl group, an ethyl group, a n-propyl group, an isopropyl group, a n-butyl group, an isobutyl group, a sec-butyl group, a tert-butyl group, a pentyl group, a hexyl group, an octyl group, a decyl group, a dodecyl group, and a benzyl group. Examples of a cycloalkyl group having 5 to 6 carbon atoms, which is another preferable alternative of R¹ and R², include, but are not limited to, a cyclopentyl group, and a cyclohexyl group.

In case that a constituent unit derived from (A1) a monoallylamine-based monomer is an addition salt, the structure is represented by the following formula (I'):

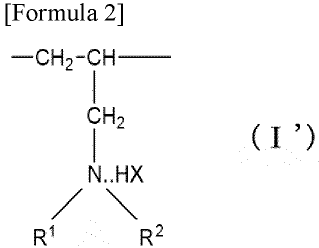

In the formula (I'), HX represents an inorganic acid or an organic acid, H represents a hydrogen atom, and X is not especially limited as long as it is a group capable of forming an inorganic acid or an organic acid together with hydrogen.

The type of the addition salt is not particularly limited, and from the viewpoints of availability, reaction controllability, and the like, for example, hydrochloride, sulfate, phosphate, nitrate, sulfite, phosphite, nitrite, hydrobromide, acetate, amidosulfate, methanesulfonate, trifluoroacetate, and p-toluenesulfonate can be used.

In particular, hydrochloride, sulfate, phosphate, and amidosulfate are preferred, and hydrochloride, sulfate, phosphate, and amidosulfate having a structure derived from monoallylamine are particularly preferred.

(a1) an allylamine (co)polymer may consist of constituent unit(s) derived from (A1) a monoallylamine-based monomer (namely, may be a homopolymer of a monoallylamine-based monomer), or may have another constituent unit in addition to a constituent unit derived from (A1) a monoallylamine-based monomer (namely, may be a copolymer of a monoallylamine-based monomer, and another monomer).

The ratio of constituent unit(s) derived from (A1) a monoallylamine-based monomer to all constituent units of (a1) an allylamine (co)polymer is preferably 5 to 100 mol%, and particularly preferably 10 to 100 mol%.

When the ratio of constituent unit(s) derived from (A1) a monoallylamine-based monomer is 5 mol% or more, advantageous properties such as high blood cell separation performance can be realized.

The ratio of constituent unit(s) derived from (A1) a monoallylamine-based monomer can be appropriately adjusted by adjusting the ratio of a monoallylamine-based monomer used in production of (a1) an allylamine (co)polymer, and other polymerization conditions.

A constituent unit other than one derived from (A1) a monoallylamine-based monomer which constitutes (a1) an allylamine (co)polymer is not particularly limited, and such a constituent unit can be derived by performing copolymerization appropriately using a monomer copolymerizable with a monoallylamine-based monomer.

Preferable examples of that copolymerizable monomer include, but are not limited to, diallylamines such as diallylamine, and diallylmethylamine, or addition salts thereof; anionic monomers such as dicarboxylic acid, and acrylamides such as acrylamide, dimethylacrylamide, acryloylmorpholine, N-[3-(dimethylamino)propyl](meth)acrylamide, (3-acrylamidopropyl)trimethylammonium chloride and (3-methacrylamidopropyl)trimethylammonium chloride; allyl alcohols and allyl ethers such as ethylene glycol monoallyl ether; allyl sulfonic acids such as (sodium) (meth)allylsulfonate, or addition salts thereof; vinyl sulfonic acids such as (sodium) vinyl sulfonate, or addition salts thereof; and sodium isoprenesulfonate.

Among these, diallylamines, anionic monomers such as dicarboxylic acid, acrylamides and the like can be preferably used. From the viewpoint of realizing favorable blood cell separation performance and the like, it is particularly preferable that diallylamines having a secondary amino group are copolymerized.

As the monomer copolymerizable with a monoallylamine-based monomer, only one type of monomer(s) can be used, or two or more types of monomers can be used in combination.

The details of diallylamines will be described below in relation to (a2) a diallylamine (co)polymer.

### Anionic Constituent Unit(s)

An anionic monomer such as dicarboxylic acid can be copolymerized to introduce anionic constituent unit(s) into (a1) an allylamine (co)polymer. When (a1) an allylamine (co)polymer has anionic constituent unit(s), (a1) the allylamine (co)polymer becomes what is called an amphoteric polymer, so that advantageous effects such as miscibility with blood cells or the like, and hydrophilicity can be realized.

Anionic constituent unit(s) of the present embodiment may be constituent unit(s) capable of having negative charge through desorption, and is not limited otherwise, and preferably has a structure derived from a divalent carboxylic acid, but it particularly preferably has a structure represented by the following structural formula (1), (2), or (3):

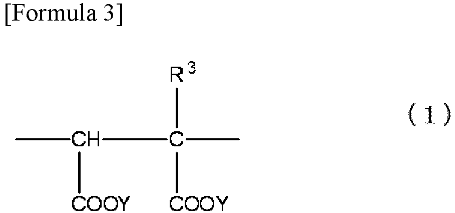

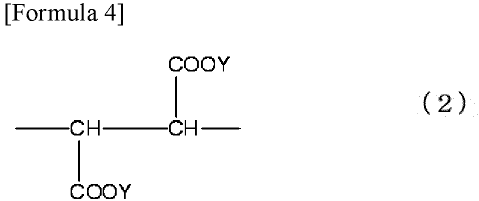

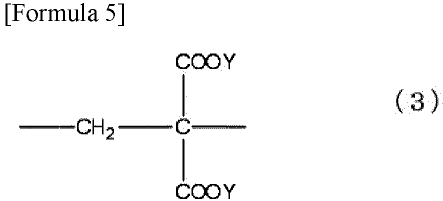

In the formula (1), R³ is hydrogen or a methyl group, and in the formulas (1), (2), and (3), Y is hydrogen, Na, K, NH₄, 1/2Ca, 1/2Mg, 1/2Fe, 1/3Al, or 1/3Fe each independently with respect to a carboxy group to be bonded.

Anionic constituent unit(s) may have a structure corresponding to none of the structural formulas (1), (2), and (3) as long as it has an anionic group such as a carboxyl group.

When Specific Polymer(s) have anionic constituent unit(s), one type of anionic constituent unit(s) may be used, or a plurality of types of anionic constituent units having different structures from each other may be used in combination.

When a plurality of types of anionic constituent units different from each other are used, the respective anionic constituent units may have different structures represented by the same general structural formula (1), (2), or (3), or may have different structures represented by different general structural formulas. In the former case, for example, a plurality of types of anionic constituent units that are represented by the general structural formula (1) but have different structures in Y from each other may be employed. In the latter case, one type of anionic constituent unit(s) having a structure represented by the structural formula (1), and another type of anionic constituent unit(s) having a structure represented by the structural formula (2) may be employed.

At least some of anionic constituent unit(s) are preferably derived from maleic acid, and in this case, the anionic constituent unit (s) has a structure represented by the formula (2), in which both of Ys are hydrogen.

When Specific Polymer(s) have anionic constituent unit(s), the content of the anionic constituent unit(s) are not particularly limited. When anionic constituent unit(s) are derived from an unsaturated dicarboxylic acid, the molar ratio thereof to constituent unit(s) derived from (A1) a monoallylamine-based monomer is such that the constituent unit(s) derived from (A1) a monoallylamine-based monomer/anionic constituent unit(s) is preferably 1/0.1 to 1/0.5, and particularly preferably 1/0.2 to 1/0.5.

At least some of anionic constituent unit(s) may be derived from an unsaturated monocarboxylic acid. When anionic constituent unit(s) are derived from an unsaturated monocarboxylic acid, the ratio of constituent unit(s) derived from (A1) a monoallylamine-based monomer/anionic constituent unit(s) is preferably 1/1 to 100/1, and particularly preferably 1/1 to 10/1.

The total ratio of constituent unit(s) derived from (A1) a monoallylamine-based monomer and anionic constituent unit(s) to all constituent units (if an anionic constituent unit is not contained, the ratio of constituent unit(s) derived from (A1) a monoallylamine-based monomer to all constituent units) is preferably 80 mol% or more, and particularly preferably 90 mol% or more. When the total ratio of constituent unit(s) derived from (A1) a monoallylamine-based monomer and anionic constituent unit(s) to all constituent units is 80 mol% or more, favorable properties such as improvement of blood cell separation ability can be realized.

The molecular weight of (a1) an allylamine (co)polymer is not particularly limited, and a (co)polymer having a preferable molecular weight according to a mode of use of a blood cell separation agent, and relationship with other components or the like may be obtained commercially or by polymerization.

From the viewpoints of ease in carrying it on an inorganic carrier, and conducting polymerization with practically allowable time and cost, a weight average molecular weight (Mw) is preferably 500 to 10,000,000. The weight average molecular weight (Mw) is more preferably 1,000 to 1,000,000. The weight average molecular weight (Mw) is particularly preferably 2,000 to 500,000.

From the viewpoint of realizing favorable blood cell separation performance and the like, the weight average molecular weight (Mw) is preferably higher in the above-described range, and for example, the weight average molecular weight (Mw) is preferably 8,000 or more.

The weight average molecular weight (Mw) of (a1) an allylamine (co)polymer can be measured, for example, with a liquid chromatograph by gel permeation chromatography (GPC method). More specifically, it can be measured by, for example, the method described in Examples herein.

The molecular weight of (a1) an allylamine (co)polymer can be appropriately adjusted by choosing the presence or absence, type and composition of comonomer(s), the temperature, time and pressure employed in the polymerization process, the type and amount of a radical initiator used in the polymerization process, and the like.

The rotational viscosity [η] of (a1) an allylamine (co)polymer is also not particularly limited, and can be appropriately set in consideration of ease of carrying it on an inorganic carrier, production cost and the like, and is preferably 10 to 700 mPa•s (25°C), and particularly preferably 10 to 60 mPa•s (25°C).

The rotational viscosity [η] can be measured by a method usually employed in the art, and for example, can be measured with a digital B type viscometer DV-3T manufactured by AMETEK Brookfield. The measurement can be performed with a ULA adapter used, representatively with a liquid amount of 16 mL and a liquid temperature of 25°C.

Also, the rotational viscosity [η] can be appropriately adjusted by adjusting or choosing diluted concentrations employed in polymerization, the presence or absence, type and composition of comonomer(s), the temperature, time and pressure employed in the polymerization process, the type and amount of a radical initiator used in the polymerization process, and the like.

### (a2) Diallylamine (Co) Polymer

(a2) a diallylamine (co)polymer, preferably used as Specific Polymer(s) of the present invention, is a (co)polymer having a constituent unit derived from (A2) a diallylamine-based monomer. A diallylamine-based monomer has an amino group, and hence a constituent unit derived from (A2) a diallylamine-based monomer corresponds to Specific Constituent Unit(s) in (a2) a diallylamine (co)polymer.

Here, the term "(co)polymer" encompasses both a homopolymer and a copolymer, and accordingly, (a2) a diallylamine (co)polymer may be a homopolymer consisting of constituent unit(s) derived from (A2) a diallylamine-based monomer, or may be a copolymer having constituent unit(s) derived from (A2) a diallylamine-based monomer and different constituent unit(s).

(a2) a diallylamine (co)polymer has constituent unit(s) derived from a diallylamine-based monomer (A2).

More specifically, constituent unit(s) derived from (A2) a diallylamine-based monomer preferably has a structure represented by the following structural formula (IIa) or (IIb), or a structure of an inorganic acid salt thereof or an organic acid salt thereof, or a structure represented by the following structural formula (IIIa) or (IIIb):

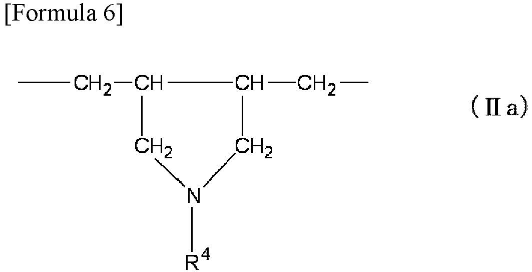

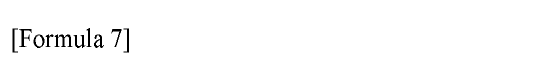

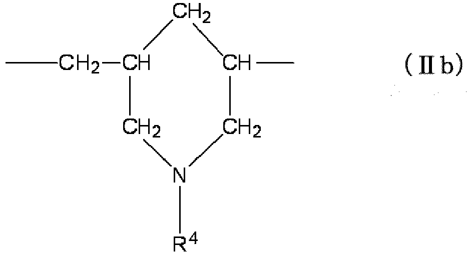

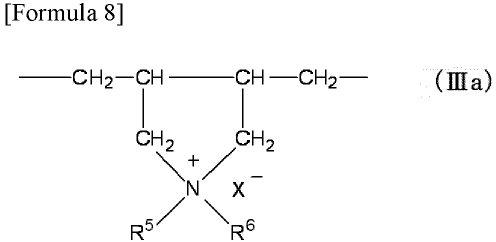

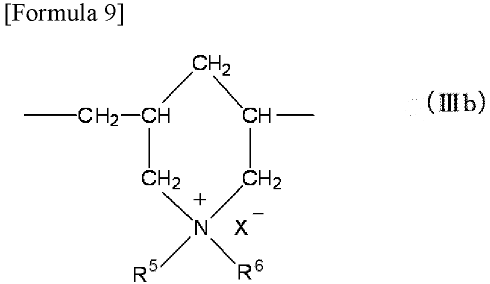

In the formulas (IIa) and (IIb), R⁴ represents a hydrogen atom, or a monovalent organic group, and is preferably a hydrogen atom, an alkyl group having 1 to 10 carbon atoms, a cycloalkyl group having 5 to 10 carbon atoms, or an aralkyl group having 7 to 10 carbon atoms. In the formula (IIIa) and (IIIb), R⁵ and R⁶ each independently represent a hydrogen atom or a monovalent organic group (at least one of R⁵ and R⁶ being a hydrogen atom), and are preferably a hydrogen atom, an alkyl group having 1 to 10 carbon atoms, a cycloalkyl group having 5 to 10 carbon atoms, or an aralkyl group having 7 to 10 carbon atoms; and X⁻ is a counter ion.

In the formulas (IIa) and (IIb), R⁴ is more preferably a hydrogen atom, a methyl group, an ethyl group, or a benzyl group, and particularly preferably a hydrogen atom, or a methyl group.

In the formulas (IIIa) and (IIIb), at least one of R⁵ and R⁶ is a hydrogen atom, and R⁵ and R⁶ are preferably each independently a hydrogen atom, a methyl group, an ethyl group, or a benzyl group, and particularly preferably a hydrogen atom, or a methyl group.

(a2) a diallylamine (co)polymer may have, as a constituent unit derived from (A2) a diallylamine-based monomer, constituent unit(s) having the structure represented by the structural formula (IIa) or (IIb), i.e., constituent unit(s) in a free structure form, or may have constituent unit(s) in the form of an inorganic acid salt or an organic acid salt represented by the formula (IIa) or (IIb), i.e. an addition salt thereof, or a structure represented by the structural formula (IIIa) or (IIIb), i.e. a structure having a counter ion.

In the production of (a2) a diallylamine (co)polymer, from the viewpoint of production cost and the like, a diallylamine monomer having an addition salt or a counter ion is preferably used. A process for removing an addition salt such as HCl and a counter ion from a polymer is complicated, and can increase the cost. Therefore, use of an addition salt type or counter ion type of (a2) a diallylamine (co)polymer, which can be produced without such a process, is a preferable embodiment also from the viewpoint of cost and the like.

From the viewpoints of availability, reaction controllability and the like, an inorganic acid salt or organic acid salt having the structure represented by the structural formula (IIa) or (IIb) is preferably a hydrochloride, carboxylate, sulfonate, or alkyl sulfate salt, and particularly preferably a hydrochloride.

When (a2) a diallylamine (co)polymer has, as constituent unit(s) derived from (A2) a diallylamine-based monomer, the structure represented by the structural formula (IIIa) or (IIIb), the counter ion X⁻ is not particularly limited, and from the viewpoints of availability, reaction controllability and the like, it is preferably a chlorine ion, a carboxylic acid ion, a sulfonic acid ion, or an alkyl sulfate ion, and particularly preferably a chlorine ion, or an ethyl sulfate ion.

In (a2) a diallylamine (co)polymer, constituent unit(s) derived from one type of (A2) diallylamine-based monomer(s) may be singly used, or constituent units derived from a plurality of types of (A2) diallylamine-based monomers having different structures from each other may be used in combination.

When constituent units respectively derived from a plurality of types of (A2) diallylamine-based monomers different from each other are used, the respective diallylamine-based constituent units may have different structures represented by the same general structural formula (IIa), (IIb), (IIIa), or (IIIb), or may have different structures represented by different general structural formulas. In the former case, for example, constituent units derived from a plurality of types of (A2) diallylamine-based monomers represented by the general structural formula (IIa) but different in the structure of R⁴ from each other may be used. In the latter case, for example, one type of constituent unit(s) (A2) having a structure represented by the structural formula (IIa) and another type of constituent unit(s) (A2) having a structure represented by the structural formula (IIIa) may be used.

The content of constituent unit(s) derived from (A2) diallylamine-based monomer(s) in (a2) a diallylamine (co)polymer is not particularly limited, and the constituent unit(s) preferably account for 1 mol% or more, and more preferably 5 to 100 mol% of all constituent units of (a2) a diallylamine (co)polymer.

The ratio of constituent unit(s) derived from (A2) diallylamine-based monomer(s) can be appropriately adjusted by adjusting the ratio of diallylamine-based monomer(s) and other polymerization conditions employed in the production of (a2) a diallylamine (co)polymer.

A constituent unit other than constituent unit(s) derived from (A2) diallylamine-based monomer(s) in (a2) diallylamine (co)polymer is not particularly limited, and such a constituent unit can be derived by performing copolymerization appropriately using a monomer copolymerizable with a diallylamine-based monomer.

Preferable examples of that copolymerizable monomer include, but are not limited to, monoallylamines, or addition salts thereof; anionic monomers such as dicarboxylic acid, and sulfur dioxide; and acrylamides such as acrylamide, dimethylacrylamide, acryloylmorpholine, N-[3-(dimethylamino)propyl](meth)acrylamide, (3-acrylamidopropyl)trimethylammonium chloride, (3-methacrylamidopropyl)trimethylammonium chloride; allyl alcohols and allyl ethers such as ethylene glycol monoallyl ether; allyl sulfonic acids such as (sodium) (meth)allylsulfonate, or addition salts thereof; vinyl sulfonic acids such as (sodium) vinyl sulfonate, or addition salts thereof; and sodium isoprenesulfonate.

Among these, monoallylamines, anionic monomers such as dicarboxylic acid, sulfur dioxide, acrylamides, and the like can be preferably used.

As for monomers other than diallylamine-based monomers, one type of monomers can be used, or two or more types of monomers can be used in combination.

The details of preferable embodiments and the like of constituent unit(s) derived from a monoallylamine, an anionic monomer such as dicarboxylic acid, or the like are the same as those described above in relation to (a1) an allylamine (co)polymer.

### Sulfur Dioxide Constituent Unit

In the present embodiment, when Specific Polymer(s) have sulfur dioxide constituent unit(s), advantageous properties such as improved polymerizability, and blood cell separation ability can be imparted to Specific Polymer(s).

Sulfur dioxide constituent unit(s) in a copolymer of the present embodiment are derived from sulfur dioxide having a structure represented by the following structural formula (4):

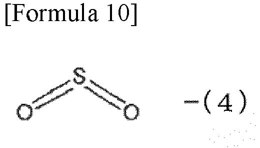

The ratio of sulfur dioxide constituent unit(s) to all constituent units of a copolymer of the present embodiment is preferably 1 mol% or more. In particular, when the ratio of sulfur dioxide-derived constituent unit(s) is 1 mol% or more, advantageous properties such as improved polymerizability, and hydrophobicity, and improved performance of separating an anionic component (such as blood cells) can be more effectively imparted to a copolymer of the present embodiment.

The ratio of sulfur dioxide constituent units is more preferably 1 to 50 mol%, and particularly preferably 10 to 50 mol%.

In the present embodiment, the ratio between constituent unit(s) derived from (A2) diallylamine-based monomer(s) and sulfur dioxide constituent unit(s) is also not particularly limited, and any ratio can be selected as long as these are copolymerizable. From the viewpoint of increasing the molecular weight of a copolymer, it is preferable that the ratios of these constituent units are not largely different from each other, and for example, the ratio between constituent unit(s) derived from (A2) diallylamine-based monomer(s) and constituent unit(s) derived from sulfur dioxide is preferably 99:1 to 50:50, and particularly preferably 90:10 to 50:50.

The molecular weight of (a2) a diallylamine (co)polymer is not particularly limited, and such a (co)polymer having a preferable molecular weight according to a mode of use of a blood cell separation agent, and relationship with other components or the like may be obtained commercially or by polymerization.

From the viewpoints of ease in carrying it on an inorganic carrier, and conducting polymerization with practically allowable time and cost, the weight average molecular weight (Mw) is preferably 500 to 10,000,000. The weight average molecular weight (Mw) is more preferably 1,000 to 1,000,000. The weight average molecular weight (Mw) is particularly preferably 2,000 to 500,000.

From the viewpoint of realizing favorable blood cell separation performance and the like, the weight average molecular weight (Mw) is preferably higher in the above-described range, and for example, the weight average molecular weight (Mw) is preferably 15,000 or more.

The weight average molecular weight (Mw) of (a2) a diallylamine (co)polymer can be measured, for example, with a liquid chromatograph by gel permeation chromatography (GPC method). More specifically, it can be measured by, for example, the method described in Examples herein.

The molecular weight of (a2) a diallylamine (co)polymer can be appropriately adjusted by choosing the presence or absence, type and composition of comonomer(s), the temperature, time and pressure employed in the polymerization process, the type and amount of a radical initiator used in the polymerization process, and the like.

The rotational viscosity [η] of (a2) a diallylamine (co)polymer is also not particularly limited, and can be appropriately set in consideration of ease of carrying it on an inorganic carrier, production cost and the like, and is preferably 10 to 1,500 mPa•s (25°C), and particularly preferably 10 to 500 mPa•s (25°C).

The rotational viscosity [η] can be measured by a method usually employed in the art, and for example, can be measured with a digital B type viscometer DV-3T manufactured by AMETEK Brookfield. The measurement can be performed with a ULA adapter, typically with a liquid amount of 16 mL at a liquid temperature of 25°C.

Also, the rotational viscosity [η] can be appropriately adjusted by choosing the presence or absence, type and composition of comonomer(s), the temperature, time and pressure employed in the polymerization process, the type and amount of a radical initiator used in the polymerization process, and the like.

### (a3) Polyethyleneimine

(a3) a polyethyleneimine, preferably used as Specific Polymer in the present invention, is a polymer having constituent(s) unit derived from ethyleneimine, and more specifically, has constituent unit(s) having a structure represented by the following formula (IVa):

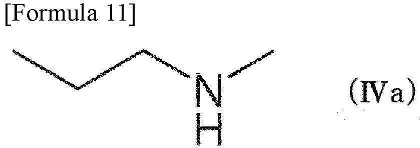

(a3) a polyethyleneimine has constituent unit(s) having the structure represented by the formula (IVa) and hence has an amino group. It therefore corresponds to a polymer having constituent(s) unit with amino group(s). i.e., Specific Polymer. The amino group(s) are a secondary amino group, and therefore, it also corresponds to a (co)polymer having constituent unit(s) with a secondary amino group or a tertiary amino group, which is preferable as Specific Polymer(s).

(a3) a polyethyleneimine may consist of constituent unit(s) having the structure represented by the formula (IVa), or may further have constituent unit(s) having another structure.

When (a3) a polyethyleneimine consists of constituent unit(s) having the structure represented by the formula (IVa), (a3) the polyethyleneimine has a linear structure.

(a3) a polyethyleneimine may have a branched structure by means of having a tertiary amino group and may have, for example, a structure represented by the following formula (IVb). Even in the structure of the following formula (IVb), a secondary amino group is present in a linear portion and a primary amino group is present at the end. It therefore corresponds to a polymer having constituent unit(s) with amino group(s), at least some of which are a primary amino group, a secondary amino group, or a tertiary amino group (i.e., Specific Polymer(s)), and also corresponds to a (co)polymer having constituent unit(s) with a secondary amino group or a tertiary amino group, which is particularly preferable as Specific Polymer.

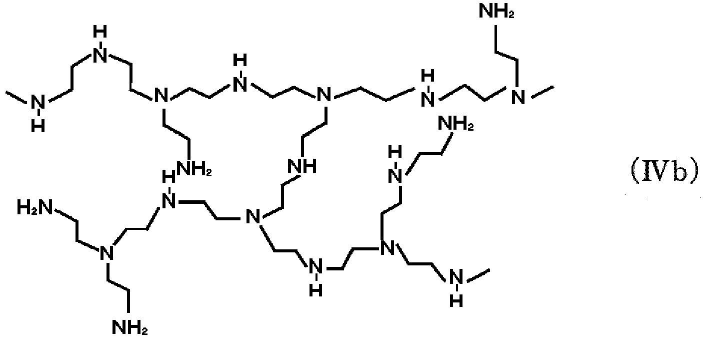

In the formulas (IVa) and (IVb), all amino groups are shown in a free form, but some or all of these amino groups may be addition salts. The type of the addition salts is not particularly limited, and as for (a1) an allylamine (co)polymer and (a2) a diallylamine (co)polymer, for example, various organic acid salts and inorganic acid salts described above can be appropriately used.

### [Formula 12]

The molecular weight of (a3) a polyethyleneimine is not particularly limited, and the weight average molecular weight is preferably in a range of 100 to 2,000,000, more preferably 500 to 2,000,000, and particularly preferably in a range of 1,000 to 2,000,000.

(a3) a polyethyleneimine can be synthesized, for example, by ring-opening polymerization of ethyleneimine in the presence of an acid catalyst.

Alternatively, a commercially available polyethyleneimine may be used and includes Lupasol commercially available from BASF (brand name: Lupasol SK (average molecular weight: about 2,000,000), Lupasol G20 (average molecular weight: about 1,300), Lupasol G20WF (average molecular weight: about 1,300), Lupasol P (average molecular weight: about 750,000), Lupasol PS (average molecular weight: about 750,000), Lupasol PR8515 (2,000), Lupasol PN40, Lupasol WF (average molecular weight: about 25,000), Lupasol SC-61B (average molecular weight: 110,000), and Lupasol FG), Epomin commercially available from Nippon Shokubai Co., Ltd. (Model No: SP-003 (average molecular weight: about 300), SP-006 (average molecular weight: about 600), SP-012 (average molecular weight: about 1,200), SP-018 (average molecular weight: about 1,800), SP-200 (average molecular weight: about 10,000), and P-1000 (average molecular weight: about 70,000)), and BPEI commercially available from Wako Pure Chemical Industries Ltd. (manufacturer code: 161-17831 (average molecular weight: about 600), manufacturer code: 167-17811 (average molecular weight: about 1,800), and manufacturer code: 164-17821 (average molecular weight: about 10,000)).

### Method for Producing Specific Polymer(s)

A method for producing Specific Polymer(s) is not particularly limited, and Specific Polymer(s) can be produced by any methods conventionally known in this technical field, and can be produced by, for example, homopolymerizing a monoallylamine or diallylamine monomer, or an inorganic acid salt or organic acid salt thereof, or copolymerizing it with another monomer if desired.

In homopolymerizing a monoallylamine or diallylamine, or an inorganic acid salt or organic acid salt thereof, or copolymerizing it with another monomer, a solvent is not particularly limited, and may be an aqueous solvent, or an organic solvent such as alcohol, ether, sulfoxide, or amide, and is preferably an aqueous solvent.

In homopolymerizing a monoallylamine or diallylamine, or an inorganic acid salt or organic acid salt thereof, or copolymerizing it with another monomer, a monomer concentration varies depending on the type of a monomer, or the type of a solvent used in the (co)polymerization, but in case of an aqueous solvent, is usually 10 to 75% by weight. This (co)polymerization reaction is usually a radical polymerization reaction, and is performed in the presence of a radical polymerization catalyst. The type of the radical polymerization catalyst is not particularly limited, and preferable examples include peroxides such as t-butyl hydroperoxide, persulfates such as ammonium persulfate, sodium persulfate, and potassium persulfate, and azobis-based or diazo-based aqueous azo compounds.

An amount of the radical polymerization catalyst to be added is generally 0.1 to 20 mol%, and preferably 1.0 to 10 mol% with respect to all monomers. A polymerization temperature is generally 0 to 100°C, and preferably 5 to 80°C, and a polymerization time is generally 1 to 150 hours, and preferably 5 to 100 hours. As a polymerization atmosphere, no serious problem occurs in polymerizability even in the air, and the polymerization can be performed in an inert gas atmosphere of nitrogen or the like.

### Inorganic Carrier

An agent for use in blood cell separation of the present invention is an agent for use in blood cell separation comprising an inorganic carrier, and polymer(s) having constituent unit(s) with amino group(s) (Specific Polymer(s)), wherein at least some of the polymer(s) are carried on the inorganic carrier, preferably by adsorption through electrostatic interaction and/or by a covalent bond.

The carried Specific Polymer(s) on an inorganic carrier is preferable from the viewpoints of handleability, reuse of the blood cell separation agent, and reduced contamination of a sample separated from blood cells with the blood cell separation agent.

The shape or form of the inorganic carrier is not particularly limited, and for example, a container, an inner wall of a tube, an inorganic particle, a magnetic bead, glass wool, or glass filter paper can be used as the inorganic carrier. The material of the inorganic carrier can be glass, silica, kaolin, zeolite, bentonite or the like because such a material itself has blood cell separation ability, and can easily carry Specific (Co) Polymer(s).

When a particulate inorganic carrier is used, the particle size of the inorganic carrier is not particularly limited, either, and a primary particle size thereof is preferably 0.05 to 500 µm, and particularly preferably 0.1 to 50 µm.

In the present invention, a method for carrying Specific Polymer(s) on an inorganic carrier is not particularly limited, and Specific Polymer(s) is preferably carried on an inorganic carrier by being adsorbed through electrostatic interaction, and/or being bonded by a covalent bond, which allows Specific Polymer(s) to be stably carried.

A method for adsorbing Specific Polymer(s) on an inorganic carrier through electrostatic interaction is not particularly limited, but can be preferably performed by adjusting a pH of a dispersion or a solution in which Specific Polymer(s) are dispersed or dissolved, adjusting concentrations of solid components, particularly, polymers, and so on.

For example, when the inorganic carrier is silica, the pH of a dispersion or a solution containing Specific Polymer(s) is preferably 5 to 11, and particularly preferably pH 7 to 10.

The concentration of Specific Polymer(s) is preferably 1.0 to 20% by weight, and particularly preferably 2.5 to 10% by weight.

A method for bonding Specific Polymer(s) to an inorganic carrier by covalent bond is also not particularly limited, and a covalent bond can be made through a reaction between a functional group such as an amino group of Specific Polymer(s) and a reactive group present on the surface of the inorganic carrier, or by using a compound having reactivity with both of them.

For example, when the inorganic carrier is silica, a silane coupling agent is preferably used. The type of the silane coupling agent is not particularly limited, and epoxy silane, methacrylic silane, acrylic silane, chloro silane, and the like can be preferably used. In particular, epoxy silane is particularly preferably used.

In those methods, Specific Polymer(s) may be crosslinked with each other. When Specific Polymer(s) are adsorbed on an inorganic carrier through electrostatic interaction, Specific Polymer(s) tends to become desorbed through pH change, but in case that Specific Polymer(s) are crosslinked with each other, even if some of Specific Polymer(s) are desorbed through pH change, the crosslinked Specific Polymers as a whole remain adsorbed. Therefore, when Specific Polymer(s) are adsorbed on an inorganic carrier by electrostatic interaction, the crosslinking of Specific Polymer(s) with each other is technically much valuable.

In the crosslinking of Specific Polymer(s) with each other, crosslinking agents conventionally used in this technical field can be appropriately used, and peroxide-based crosslinking agents, epoxy-based crosslinking agents, alkyl halide-based crosslinking agents, and the like can be appropriately used. In particular, the crosslinking is performed preferably using a crosslinking agent that is a compound containing, in total, at least two functional groups capable of forming a covalent bond with an amino group of Specific Polymer(s). Examples of such a functional group include a halogen group, an aldehyde group, an epoxy group, a carboxyl group, an acid anhydride group, an acid halide group, a N-chloroformyl group, a chloroformate group, an imide ether group, an amidinyl group, an isocyanate group, and a vinyl group.

Specific examples of the crosslinking agent include ethylene glycol diacrylate, propylene glycol diacrylate, butylene glycol diacrylate, ethylene glycol dimethacrylate, propylene glycol dimethacrylate, butylene glycol dimethacrylate, polyethylene glycol dimethacrylate, polyethylene glycol diacrylate, methylene bisacrylamide, methylene bismethacrylamide, ethylene bisacrylamide, epichlorohydrin, toluene diisocyanate, ethylene bismethacrylamide, ethylidene bisacrylamide, divinyl benzene, bisphenol A dimethacrylate, bisphenol A diacrylate, 1,4-butanediol diglycidyl ether, 1,2-ethanediol diglycidyl ether (ethylene glycol diglycidyl ether (EGDGE)), 1,3-dichloropropane, 1,2-dichloroethane, 1,3-dibromopropane, 1,2-dibromoethane, succinyl dichloride, dimethyl succinate, acryloyl chloride, and pyromellitic dianhydride. In particular, epichlorohydrin, 1,3-dichloropropane, and ethylene glycol diglycidyl ether (EGDGE) can be particularly preferably used.

The concentration of a crosslinking agent in crosslinking Specific Polymer(s) with each other is such that the molar ratio of a crosslinking agent/Specific Polymer(s) is preferably 0.05 to 20, and particularly preferably 0.4 to 15.

When the amount of the crosslinking agent falls in either of the preferable ranges, the blood cell separation performance can be improved in maintaining the adsorption of Specific Polymer(s) as a whole.

### Method of Separating Blood Cells

When the agent for use in blood cell separation described above is contacted with a specimen containing blood cells, the blood cells are separated, so that the blood cells and a liquid phase can be separated from each other. Accordingly, a method of separating blood cells of the present invention is not particularly limited except for this point and allow blood cells to be separated in various forms.

For example, in case of a blood cell separation agent wherein Specific Polymer(s) are carried on a particulate inorganic carrier, the blood cell separation agent may be added to and mixed with a sample containing blood cells in a collection container, or a collected specimen containing blood cells may be added to and mixed with the blood cell separation agent in a container, so that the blood cells are captured, and the sample can be separated into the blood cells and a liquid phase. This method can be easily employed in clinical practice and advantageously versatile.

In a blood cell separation agent of the present invention, Specific Polymer(s) are carried on an inorganic carrier, and therefore, a liquid phase separated from blood cells is not contaminated with the blood cell separation agent. In addition, even when a sample is obtained under conditions where the blood cell separation agent is exposed to an acidic solution or an alkaline solution, Specific Polymer(s) are not desorbed from the inorganic carrier of the blood cell separation agent, and hence the sample is not contaminated with Specific Polymer(s). Therefore, a sample having a pH suitable for pH conditions of an analysis system, such as the pH of a mobile phase of HPLC, can be obtained, and such a sample can be directly subjected to an analysis even if the analysis requires a sample having a prescribed pH.

In case of a blood cell separation agent wherein Specific Polymer(s) are carried on the surface of an inner wall of a sample collection tube, blood cells can be separated only by putting a sample in the collection tube and mixing the resultant by inversion if appropriate. Therefore, this method is highly practical and has a merit that the sample collection tube on which Specific Polymer(s) are carried can be repeatedly used. A liquid phase separated from blood cells is not contaminated with the blood cell separation agent, and particularly when a sample is obtained by blood cell separation under conditions of exposure to an acidic solution or an alkaline solution, Specific Polymer(s) are not desorbed from the inorganic carrier, and a sample having a pH suitable for pH conditions of an analysis system can be obtained in the same manner as in a blood cell separation agent in which Specific Polymer(s) are carried on a particulate or fibrous inorganic carrier.

A blood cell separation agent in which Specific Polymer(s) are carried on a particulate inorganic carrier can be filled in a container such as a column or a sample flow path of an analyzer, together with a support composed of a filtering material if appropriate, to construct a blood cell separation member or a blood cell separation part. Alternatively, a blood cell separation agent in which Specific Polymer(s) are carried on a particulate inorganic carrier can be first mixed with a sample containing blood cells, and the resultant mixture is then introduced into a container, such as a column, having been filled with a filtering material, to separate the blood cells and a liquid phase. In these embodiments, a sample which has flowed out of the blood cell separation member or the blood cell separation part can be directly subjected to various analyses, and hence those methods are applicable to a case of requiring a sample having a pH suitable for pH conditions of an analysis system, such as the pH of a mobile phase of HPLC. Therefore, various analyses can be continuously performed following the blood cell separation. Examples of the filtering material contained in the support include sheet-shaped or particulate filter paper, sheet-shaped or particulate chromatography paper, a particulate silica gel, a particulate or fibrous alumina, particulate diatomite, particulate or fibrous glass, and a combination thereof.

Similarly, a blood cell separation agent in which Specific Polymer(s) are carried on a sheet-shaped or fibrous inorganic carrier may be filled in a container, such as a column or a sample flow path of an analyzer, to construct a blood cell separation member or a blood cell separation part.

A blood cell separation agent and method of the present invention are applicable to any sample containing blood cells, and can be thus applied to, for example, blood, urine, lymph, a brain fluid, a body cavity fluid, an exudate fluid, digestive juices, and feces. A representative applicable sample is blood, which encompasses a whole blood specimen, and a blood specimen containing an anticoagulant such as heparin, and a whole blood specimen is the most representative sample.

### Examples

The present invention will now be further specifically described with reference to Examples, and it is to be noted that the present invention is not limited to these.

### (Method for Measuring Weight Average Molecular Weight)

The weight average molecular weights of the following cationic polymers were measured by the following methods, respectively.

The weight average molecular weight (Mw) of a cationic polymer containing an anionic constituent unit (ii) (Example 11) was measured with Hitachi L-2000 High Performance Liquid Chromatograph by gel permeation chromatography (GPC method).

As an eluent flow path pump, Hitachi L-2130 pump was used, as a detector, Hitachi L-2490 RI detector was used, and as columns, two TOSOH TSKgel α-M (exclusion limit molecular weight: 10,000,000) connected in series were used. A sample was prepared to a concentration of 0.5 g/100 mL with an eluent, and was used in an amount of 100 µL. As the eluent, 0.15 mol/L sodium sulfate and 1 wt% acetic acid aqueous solution were used. The measurement was performed at a column temperature of 40°C at a flow rate of 1.0 mL/min. A calibration curve was obtained by using, as a standard substance, pullulan having a molecular weight of 5,900, 47,300, 212,000, and 788,000, and the weight average molecular weight (Mw) of the cationic polymer was obtained based on the calibration curve.

The weight average molecular weights (Mw) of other cationic polymers were measured with Chromaster(R) 5450 High Performance Liquid Chromatograph manufactured by Hitachi High-Tech Corporation by gel permeation chromatography (GPC method).

As a detector, an RI refractive index detector was used, and as columns, aqueous gel filtration type columns GS-220HQ (exclusion limit molecular weight: 3,000) and GS-620HQ (exclusion limit molecular weight: 2,000,000) manufactured by Shodex Asahipak connected in series were used. A sample was prepared to a concentration of 0.5 g/100 mL with an eluent, and was used in an amount of 20 µL. As the eluent, 0.4 mol/L sodium chloride aqueous solution was used. The measurement was performed at a column temperature of 30° at a flow rate of 1.0 mL/min. A calibration curve was obtained by using, as a standard substance, polyethylene glycol having a molecular weight of 106, 194, 440, 600, 1,470, 4,100, 7,100, 10,300, 12,600, 23,000 and the like, and the weight average molecular weight (Mw) of the cationic polymer was obtained based on the calibration curve.

### 1. Preparation of Blood Cell Separation Agent

### Preparation of Cationic Polymer Immobilized on Silica Beads by Multipoint Electrostatic Interaction

### (Example 1)

To 25 mg of silica beads (manufactured by Micromod Partikeltechnologie) with a particle size of 0.3 µm, 500 µL of NaOH adjusted to 1N was added and stirred for 30 minutes. After removing the supernatant, the resultant was repeatedly washed with distilled water until neutral.

On the other hand, a diallylamine hydrochloride-sulfur dioxide copolymer (brand name: PAS-92, manufactured by Nittobo Medical Co., Ltd., class of amine: secondary, pH of polymer: 7, weight average molecular weight: 5,000, unit molecular weight with respect to amine: 197.68) was dissolved in pure water to prepare a polymer solution at a solid content concentration of 10% by weight (0.253 mmol) and adjusted with 4 N NaOH to pH 7.0.

Subsequently, epichlorohydrin (manufactured by Tokyo Chemical Industry Co., Ltd.) was added to the polymer solution at a final concentration of 3% by volume (0.19 mmol), and 500 µL of the resultant solution (molar ratio of the crosslinking agent to 1 mol of the polymer: 0.756) was mixed with the washed silica beads, and the resultant was stirred at room temperature for 1 day to carry the cationic polymer on the silica beads.

The silica beads on which the cationic polymer was immobilized was washed with MES-HEPES buffer (20 mM MES, 20 mM HEPES, pH 5.0), and subsequently with HEPES buffer (20 mM HEPES, 150 mM NaCl, pH 7.0). Thereafter, the cationic polymer-immobilized silica beads were suspended in 500 µL of HEPES buffer to be used as a blood cell separation agent.

### (Example 2)

A blood cell separation agent was prepared in the same manner as in Example 1 except that ethylene glycol diglycidyl ether (EGDGE, manufactured by Tokyo Chemical Industry Co., Ltd.) was added to the polymer solution at a final concentration of 3% by volume (0.19 mmol), and that 500 µL of the resultant solution (molar ratio of the crosslinking agent to 1 mol of the polymer: 0.381) was mixed with the washed silica beads.

### (Example 3)

A blood cell separation agent was prepared in the same manner as in Example 1 except that 25 mg of silica beads (manufactured by Micromod Partikeltechnologie) with a particle size of 0.1 µm were used.

### (Example 4)

A blood cell separation agent was prepared in the same manner as in Example 1 except that 25 mg of silica beads (manufactured by Micromod Partikeltechnologie) with a particle size of 0.5 µm were used.

### (Comparative Example 1)

25 mg of silica beads (manufactured by Micromod Partikeltechnologie) with a particle size of 0.3 µm were washed in the same manner as in Example 1, and then suspended in 500 µL of HEPES buffer without immobilizing the cationic polymer thereon, and the resultant was used as a blood cell separation agent.

### (Comparative Example 2)

A blood cell separation agent was prepared in the same manner as in Example 1 except that 500 µL of the polymer solution not containing epichlorohydrin (manufactured by Tokyo Chemical Industry Co., Ltd.) was mixed with the washed silica beads, and the resultant was stirred at room temperature for 1 day to carry the cationic polymer on the silica beads.

The preparation of the blood cell separation agents of Examples 1 to 4 and Comparative Examples 1 and 2 are all summarized in the following:

**[Table 1]**

| | Example | | | |
|---|---|---|---|---|
| | 1 | 2 | 3 | 4 |
| Carrier | Silica Beads | | | |
| Particle Size of Carrier (µm) | 0. 3 | 0. 3 | 0. 1 | 0. 5 |
| Cationic Polymer | Diallylamine Hydrochloride-Sulfur Dioxide Copolymer (PAS-92) | | | |
| Class of Amine | Secondary | | | |
| Weight Average Molecular Weight | 5, 000 | | | |
| Unit Molecular Weight with respect to Amine | 197. 68 | | | |
| pH of Polymer | 7 | | | |
| Polymer Concentration (wt%) | 10 | | | |
| Bonding Method | Electrostatic Interaction | | | |
| Crosslinking Agent | Epichlorohydrin | EGDGE | Epichlorohydrin | |
| Crosslinking Agent Concentration (vol%) | 3 | 3 | 3 | 3 |
| Crosslinking Agent/Polymer (molar ratio) | 0. 756 | 0. 381 | 0. 756 | |

**[Table 2]**

| | Comparative Example | |
|---|---|---|
| | 1 | 2 |
| Carrier | Silica Beads | |
| Particle Size of Carrier (µm) | 0. 3 | |
| Cationic Polymer | - | Diallylamine Hydrochloride-Sulfur Dioxide Copolymer (PAS-92) |
| Class of Amine | - | Secondary |
| Weight Average Molecular Weight | - | 5, 000 |
| Unit Molecular Weight with respect to Amine | - | 197. 68 |
| pH of Polymer | - | 7 |
| Polymer Concentration (wt%) | - | 10 |
| Bonding Method | - | Electrostatic Interaction |
| Crosslinking Agent | - | - |
| Crosslinking Agent Concentration (vol%) | - | - |
| Crosslinking Agent/Polymer (molar ratio) | - | - |

### Preparation of Various Cationic Polymers Immobilized on Glass Beads through Multipoint Electrostatic Interaction

### (Example 5)

To 5.0 g of glass beads having a median particle size of 53 to 38 µm (Fuji Glass Beads FGB-320, manufactured by Fuji Manufacturing Co., Ltd.), 5 mL of NaOH adjusted to 1 N was added and stirred for 30 minutes. After removing the supernatant, the resultant was repeatedly washed with distilled water until neutral.

On the other hand, an allylamine polymer (brand name: PAA-08, manufactured by Nittobo Medical Co., Ltd., class of amine: primary, pH of polymer: 7, weight average molecular weight: 8,000, unit molecular weight with respect to amine: 57.09) was dissolved in pure water at a concentration of 10% by weight (0.876 mmol) to prepare a polymer solution.

To the polymer solution, epichlorohydrin (manufactured by Tokyo Chemical Industry Co., Ltd.) was added at a final concentration of 3% by volume (0.19 mmol), and 5 mL of the resultant solution (molar ratio of the crosslinking agent to 1 mol of the polymer: 0.218) was mixed with the glass beads, and the resultant was stirred at room temperature for 4 days to immobilize the cationic polymer on the glass beads. The glass beads on which the cationic polymer was immobilized was washed with MES-HEPES buffer (20 mM MES, 20 mM HEPES, pH 5.0), and subsequently with HEPES buffer (20 mM HEPES, 150 mM NaCl, pH 7.0). Thereafter, the cationic polymer-immobilized glass beads were suspended in 5 mL of HEPES buffer to be used as a blood cell separation agent.

### (Example 6)

A blood cell separation agent was prepared in the same manner as in Example 5 except that a polymer solution was prepared by dissolving, in pure water, an allylamine hydrochloride-diallylamine hydrochloride copolymer (brand name: PAA-D11-HC1, manufactured by Nittobo Medical Co., Ltd., class of amine: primary + secondary, pH of polymer: 7, weight average molecular weight: 100,000, unit molecular weight with respect to amine: 113.59) at a concentration of 10% by weight (4.402 mmol), and that 5 mL of a solution obtained by adding epichlorohydrin (manufactured by Tokyo Chemical Industry Co., Ltd.) to the polymer solution at a final concentration of 3% by volume (1.91 mmol) (molar ratio of the crosslinking agent to 1 mol of the polymer: 0.435) was mixed with the glass beads.

### (Example 7)

A blood cell separation agent was prepared in the same manner as in Example 5 except that a polymer solution was prepared by dissolving, in pure water, an allylamine hydrochloride-diallylamine hydrochloride copolymer (brand name: PAA-D19-HC1, manufactured by Nittobo Medical Co., Ltd., class of amine: primary + secondary, pH of polymer: 7, weight average molecular weight: 40,000, unit molecular weight with respect to amine: 131.62) at a concentration of 10% by weight (3.799 mmol), and that 5 mL of a solution obtained by adding epichlorohydrin (manufactured by Tokyo Chemical Industry Co., Ltd.) to the polymer solution at a final concentration of 3% by volume (1.91 mmol) (molar ratio of the crosslinking agent to 1 mol of the polymer: 0.504) was mixed with the glass beads.

### (Example 8)

A blood cell separation agent was prepared in the same manner as in Example 5 except that a polymer solution was prepared by dissolving, in pure water, a diallylamine polymer (brand name: PAS-21, manufactured by Nittobo Medical Co., Ltd., class of amine: secondary, pH of polymer: 7, weight average molecular weight: 5,000, unit molecular weight with respect to amine: 97.16) at a concentration of 10% by weight (5.146 mmol), and that 5 mL of a solution obtained by adding epichlorohydrin (manufactured by Tokyo Chemical Industry Co., Ltd.) to the polymer solution at a final concentration of 3% by volume (1.91 mmol) (molar ratio of the crosslinking agent to 1 mol of the polymer: 0.372) was mixed with the glass beads.

### (Example 9)

A blood cell separation agent was prepared in the same manner as in Example 5 except that a polymer solution was prepared by dissolving, in pure water, a diallylamine hydrochloride polymer (brand name: PAS-21CL, manufactured by Nittobo Medical Co., Ltd., class of amine: secondary, pH of polymer: 7, weight average molecular weight: 50,000, unit molecular weight with respect to amine: 133.62) in a concentration of 10% by weight (3.742 mmol), and that 5 mL of a solution obtained by adding epichlorohydrin (manufactured by Tokyo Chemical Industry Co., Ltd.) to the polymer solution at a final concentration of 3% by volume (1.91 mmol) (molar ratio of the crosslinking agent to 1 mol of the polymer: 0.511) was mixed with the glass beads.

### (Example 10)

A blood cell separation agent was prepared in the same manner as in Example 5 except that a polymer solution was prepared by dissolving, in pure water, a methyl diallylamine hydrochloride polymer (brand name: PAS-M-1L, manufactured by Nittobo Medical Co., Ltd., class of amine: tertiary, pH of polymer: 9, weight average molecular weight: 5,000, unit molecular weight with respect to amine: 147.65) in a concentration of 10% by weight (67.728 mmol), and that 5 mL of a solution obtained by adding 1,3-dichloropropane (manufactured by Tokyo Chemical Industry Co., Ltd.) to the polymer solution at a final concentration of 3% by volume (31.60 mmol) (molar ratio of the crosslinking agent to 1 mol of the polymer: 0.467) was mixed with the glass beads.

### (Example 11)

A blood cell separation agent was prepared in the same manner as in Example 5 except that a polymer solution was prepared by dissolving, in pure water, a diallylamine hydrochloride-maleic acid copolymer (brand name: PAS-411, manufactured by Nittobo Medical Co., Ltd., class of amine: secondary, pH of polymer: 7, weight average molecular weight: 40,000, unit molecular weight with respect to amine: 172.31) in a concentration of 10% by weight (2.902 mmol), and that 5 mL of a solution obtained by adding epichlorohydrin (manufactured by Tokyo Chemical Industry Co., Ltd.) to the polymer solution at a final concentration of 3% by volume (1.91 mmol) (molar ratio of the crosslinking agent to 1 mol of the polymer: 0.659) was mixed with the glass beads.

### (Example 12)

A blood cell separation agent was prepared in the same manner as in Example 5 except that a polymer solution was prepared by dissolving, in pure water, a diallylamine hydrochloride-sulfur dioxide copolymer (brand name: PAS-92, manufactured by Nittobo Medical Co., Ltd., class of amine: secondary, pH of polymer: 9, weight average molecular weight: 5,000, unit molecular weight with respect to amine: 197.68) in a concentration of 10% by weight (2.529 mmol), and that 5 mL of a solution obtained by adding epichlorohydrin (manufactured by Tokyo Chemical Industry Co., Ltd.) to the polymer solution at a final concentration of 3% by volume (1.91 mmol) (molar ratio of the crosslinking agent to 1 mol of the polymer: 0.756) was mixed with the glass beads.

### (Example 13)

A blood cell separation agent was prepared in the same manner as in Example 5 except that a polymer solution was prepared by dissolving, in pure water, a diallylamine hydrochloride-sulfur dioxide copolymer (brand name: PAS-92, manufactured by Nittobo Medical Co., Ltd., class of amine: secondary, pH of polymer: 7, weight average molecular weight: 5,000, unit molecular weight with respect to amine: 197.68) in a concentration of 2.5% by weight (0.632 mmol), and that 5 mL of a solution obtained by adding epichlorohydrin (manufactured by Tokyo Chemical Industry Co., Ltd.) to the polymer solution at a final concentration of 12.5% by volume (7.97 mmol) (molar ratio of the crosslinking agent to 1 mol of the polymer: 12.606) was mixed with the glass beads.

### (Comparative Example 3)

5.0 g of glass beads having a median particle size of 53 to 38 µm (Fuji Glass Beads FGB-320, manufactured by Fuji Manufacturing Co., Ltd.) were washed in the same manner as in Example 5, and then suspended in 5 mL of HEPES buffer without immobilizing the cationic polymer thereon, and the resultant was used as a blood cell separation agent.

The preparation of the agents for use in blood cell separation of Examples 5 to 13 and Comparative Example 3 are all summarized in the following:

**[Table 3]**

| | Example 5 | Example 6 | Example 7 | Example 8 | Example 9 |
|---|---|---|---|---|---|
| Carrier | Glass Beads | | | | |
| Particle Size of Carrier | Median Particle Size of 53 to 38 µm | | | | |
| Cationic Polymer | Allylamine Polymer (PAA-08) | Allylamine Hydrochloride-Diallylamine Hydrochloride Copolymer (PAA-D11-HCl) | Allylamine Hydrochloride-Diallylamine Hydrochloride Copolymer (PAA-D19-HCl) | Diallylamine Polymer (PAS-21) | Diallylamine Hydrochloride Polymer (PAS-21CL) |
| Weight Average Molecular Weight | 8, 000 | 100, 000 | 40, 000 | 5, 000 | 50, 000 |
| Unit Molecular Weight with respect to Amine | 57. 09 | 113. 59 | 131. 62 | 97. 16 | 133. 62 |
| Class of Amine | Primary | Primary + Secondary | Primary + Secondary | Secondary | Secondary |
| pH of Polymer | 7 | 7 | 7 | 7 | 7 |
| Polymer Concentration (wt%) | 10 | 10 | 10 | 10 | 10 |
| Crosslinking Agent | Epichlorohydrin | | | | |
| Crosslinking Agent Concentration (vol%) | 3 | 3 | 3 | 3 | 3 |
| Crosslinking Agent/Polymer (molar ratio) | 0. 218 | 0. 435 | 0. 504 | 0. 372 | 0. 511 |

**[Table 4]**

| | Example 10 | Example 11 | Example 12 | Example 13 | Comparative Example 3 |
|---|---|---|---|---|---|
| Carrier | Glass Beads | | | | |
| Particle Size of Carrier | Median Particle Size of 53 to 38 µm | | | | |
| Cationic Polymer | Methyl Diallylamine Hydrochloride Polymer (PAS-M-IL) | Diallylamine Hydrochloride-Maleic Acid Copolymer (PAS-411) | Diallylamine Hydrochloride-Sulfur Dioxide Copolymer (PAS-92) | Diallylamine Hydrochloride-Sulfur Dioxide Copolymer (PAS-92) | - |
| Weight Average Molecular Weight | 5, 000 | 40, 000 | 5, 000 | 5, 000 | - |
| Unit Molecular Weight with respect to Amine | 147. 65 | 172. 31 | 197. 68 | 197. 68 | - |
| Class of Amine | Tertiary | Secondary | Secondary | Secondary | - |
| pH of Polymer | 9 | 7 | 9 | 7 | - |
| Polymer Concentration (wt%) | 10 | 10 | 10 | 2. 5 | - |
| Crosslinking Agent | 1,3-Dichloropropane | Epichlorohydrin | | | - |
| Crosslinking Agent Concentration (vol%) | 3 | 3 | 3 | 12. 5 | - |
| Crosslinking Agent/Polymer (molar ratio) | 0. 467 | 0. 659 | 0. 756 | 12. 606 | - |

### Preparation of Cationic Polymer Immobilized on Silica Beads by Silane Coupling

### (Example 14)

To an acetic acid solution adjusted to 1.0% by volume, acrylic silane: KBM-5103 (manufactured by Shin-Etsu Chemical Co., Ltd.) was added at a final concentration of 0.1% by volume, and the resultant was stirred overnight to prepare a silane coupling agent-containing acetic acid solution. 25 mg of silica beads with a particle size of 1.5 µm (manufactured by Micromod Partikeltechnologie) were added to 500 µL of the silane coupling agent-containing acetic acid solution, the resultant was stirred at room temperature for 1 hour to cause coupling of the silane coupling agent, a supernatant was removed, and the resultant was washed with 500 µL of anhydrous ethanol, and dried in a draft.

On the other hand, a diallylamine hydrochloride-sulfur dioxide copolymer (brand name: PAS-92, manufactured by Nittobo Medical Co., Ltd., class of amine: secondary, pH of polymer: 7, weight average molecular weight: 5,000, unit molecular weight with respect to amine: 197.68) was dissolved in pure water to prepare a polymer solution at a concentration of 10% by weight (0.253 mmol) and adjusted with 4 N NaOH to pH 7.0.

Subsequently, epichlorohydrin (manufactured by Tokyo Chemical Industry Co., Ltd.) was added to the polymer solution at a final concentration of 3% by volume (0.19 mmol), and to 500 µL of the resultant solution (molar ratio of the crosslinking agent to 1 mol of the polymer: 0.756), the silica beads having been coupled to the silane coupling agent were added, and the resultant was stirred at room temperature overnight to synthesize cationic polymer-immobilized silica beads. The synthesized cationic polymer-immobilized silica beads were washed with MES-HEPES buffer (20 mM MES, 20 mM HEPES, pH 5.0), and subsequently with HEPES buffer (20 mM HEPES, 150 mM NaCl, pH 7.0). Thereafter, the cationic polymer-immobilized silica beads were suspended in 500 µL of HEPES buffer to be used as a blood cell separation agent.

### (Example 15)

A blood cell separation agent was prepared in the same manner as in Example 14 except that a silane coupling agent-containing acetic acid solution was prepared by adding, to an acetic acid solution adjusted to 1.0% by volume, epoxy silane: Z6040 (manufactured by Dow Corning Toray Co., Ltd.) at a final concentration of 0.1% by volume, and stirring the resultant overnight.

The preparation of the blood cell separation agents of Examples 14 and 15 are all summarized in the following:

**[Table 5]**

| | Example | |
|---|---|---|
| | 14 | 15 |
| Carrier | Silica Beads | |
| Particle Size of Carrier (µm) | 1. 5 | |
| Cationic Polymer | Diallylamine Hydrochloride-Sulfur Dioxide Copolymer (PAS-92) | |
| Class of Amine | Secondary | |
| Weight Average Molecular Weight | 5, 000 | |
| Unit Molecular Weight with respect to Amine | 197. 68 | |
| pH of Polymer | 7 | |
| Polymer Concentration (wt%) | 10 | |
| Bonding Method | Covalent Bond | |
| Crosslinking Agent | Epichlorohydrin | |
| Crosslinking Agent Concentration (vol%) | 3 | |
| Crosslinking Agent/Polymer (molar ratio) | 0. 756 | |
| Silane Coupling Agent | Acrylic Silane | Epoxy Silane |

### Blood Cell Separation with Cationic Polymer-immobilized Silica Beads

A column for blood cell separation was produced by placing filter paper having a retention particle size of 1 µm (manufactured by Kiriyama glass Co.) on the bottom of InertSep(R) empty reservoir (manufactured by GL Sciences Inc.) having an inner diameter of 5.7 mm and a volume of 1 mL, and filling thereon 0.1 g of a filter paper powder (manufactured by ADVANTEC) having a mesh size of 300 or more.

To 100 µL of the cationic polymer-immobilized silica beads prepared in each of Examples 1 to 4, 14, and 15, and Comparative Example 2, 5 µL of a whole blood specimen was added by pipetting to be mixed, and the entire amount of the resultant mixture was applied to the column for blood cell separation. Subsequently, the column for blood cell separation was connected to a syringe pump (manufactured by Tokyo Garasu Kikai Co., Ltd.), and 2 mL of HEPES buffer (20 mM HEPES, 150 mM NaCl, pH 7.0) was fed at a flow rate of 0.5 mL/min. As a control, a mixture of the silica beads not sensitized with the cationic polymer prepared in Comparative Example 1 and blood was applied to the column. The blood cell separation ability was visually determined in accordance with the following criteria:
O: A thickness of a blood cell layer was in the range of 1/4 or less from the top of the support, and all the blood cells were captured in the column.
△: A thickness of a blood cell layer was in a range over 1/4 from the top of the support, and all the blood cells were captured in the column.
×: Blood cells were leaked out of the column.

### Blood Cell Separation with Cationic Polymer-immobilized Glass Beads

A column for blood cell separation was produced by placing filter paper having a retention particle size of 1 µm (manufactured by Kiriyama glass Co.) on the bottom of InertSep(R) empty reservoir (manufactured by GL Sciences Inc.) having an inner diameter of 5.7 mm and a volume of 1 mL, and filling thereon 500 µL of the cationic polymer-immobilized glass beads prepared in each of Examples 5 to 13. After applying 5 µL of a whole blood specimen to the column for blood cell separation, the column for blood cell separation was connected to a syringe pump (manufactured by Tokyo Garasu Kikai Co., Ltd.), and 2 mL of HEPES buffer (20 mM HEPES, 150 mM NaCl, pH 7.0) was fed at a flow rate of 0.5 mL/min. As a control, a column filled with the glass beads not sensitized with the cationic polymer prepared in Comparative Example 3 was used. The blood cell separation ability was visually determined in accordance with the following criteria:
O: A thickness of a blood cell layer was in a range of 1/4 or less from the top of the support, and all the blood cells were captured in the column.
△: A thickness of a blood cell layer was in a range over 1/4 from the top of the support, and all the blood cells were captured in the column.
×: Blood cells were leaked out of the column.

### Desorption of Polymer from Carrier through pH Change

In a 1.5 mL tube, the blood cell separation agent of each of Examples 1 to 15 and Comparative Examples 1 and 2 was dispensed in an amount of 50 µL, a supernatant was removed by centrifugation operation, and the resultant was washed with 50 µL of HEPES buffer (20 mM HEPES, 150 mM NaCl, pH 7.0) to collect a supernatant. Thereafter, the resultant was washed again with 50 µL of MES-HEPES buffer (20 mM MES, 20 mM HEPES, pH 5.0) to similarly collect a supernatant.

1 µL of the collected supernatant was spot-applied to nitrocellulose membrane IAB075 (manufactured by ADVANTEC), and the resultant was dried at 50°C for 30 minutes, and then stained for 5 minutes with Ponceau S staining solution (manufactured by Beacle, Inc.), which is capable of coloring a cationic amino group red. Thereafter, the resultant was decolorized with a 1 vol% acetic acid solution for 5 minutes.

The results from evaluation of the blood cell separation agents of the respective Examples and Comparative Examples for the blood cell separation ability and desorption of the polymer from the carrier through pH change are shown in Tables 6 to 9 and Figs. 1 to 3.

**[Table 6]**

| | Example | | | | Comparative Example | |
|---|---|---|---|---|---|---|
| | 1 | 2 | 3 | 4 | 1 | 2 |
| Carrier | Silica Beads | | | | | |
| Particle Size of Carrier (µm) | 0. 3 | 0. 3 | 0. 1 | 0. 5 | 0. 3 | 0. 3 |
| Cationic Polymer | PAS-92 | | | | - | PAS-92 |
| Class of Amine | Secondary | | | | - | Secondary |
| Weight Average Molecular Weight | 5, 000 | | | | - | 5, 000 |
| Unit Molecular Weight with respect to Amine | 197. 68 | | | | - | 197. 68 |
| pH of Polymer | 7 | | | | - | 7 |
| Polymer Concentration (wt%) | 10 | | | | - | 10 |
| Bonding Method | Electrostatic Interaction | | | | - | Electrostatic Interaction |
| Crosslinking Agent | Epichlorohydrin | EGDGE | Epichlorohydrin | | - | - |
| Crosslinking Agent Concentration (vol%) | 3 | 3 | 3 | 3 | - | - |
| Crosslinking Agent/Polymer (molar ratio) | 0. 756 | 0. 381 | 0. 756 | | - | - |
| Silane Coupling Agent | - | - | - | - | - | - |
| Blood Cell Separation Ability | ○ | ○ | ○ | ○ | × | Δ |
| Desorption of Polymer through pH Change | not desorbed | not desorbed | not desorbed | not desorbed | - | desorbed |

**[Table 7]**

| | Example | | | | |
|---|---|---|---|---|---|
| | 5 | 6 | 7 | 8 | 9 |
| Carrier | Glass Beads | | | | |
| Particle Size of Carrier (µm) | Median Particle Size of 53 to 38 | | | | |
| Cationic Polymer | PAA-08 | PAA-D11 -HCl | PAA-D19 -HCl | PAS-21 | PAS-21CL |
| Class of Amine | Primary | Primary + Secondary | Primary + Secondary | Secondary | Secondary |
| Weight Average Molecular Weight | 8, 000 | 100, 000 | 40, 000 | 5, 000 | 50, 000 |
| Unit Molecular Weight with respect to Amine | 57. 09 | 113. 59 | 131. 62 | 97. 16 | 133. 62 |
| pH of Polymer | 7 | | | | |
| Polymer Concentration (wt%) | 10 | | | | |
| Bonding Method | Electrostatic Interaction | | | | |
| Crosslinking Agent | Epichlorohydrin | | | | |
| Crosslinking Agent Concentration (vol%) | 3 | | | | |
| Crosslinking Agent/Polymer (molar ratio) | 0. 218 | 0. 435 | 0. 504 | 0. 372 | 0. 511 |
| Silane Coupling Agent | - | - | - | - | - |
| Blood Cell Separation Ability | Δ | ○ | ○ | Δ | ○ |
| Desorption of Polymer through pH Change | not desorbed | not desorbed | not desorbed | not desorbed | not desorbed |

**[Table 8]**

| | Example | | | | Comparative Example |
|---|---|---|---|---|---|
| | 10 | 11 | 12 | 13 | 3 |
| Carrier | Glass Beads | | | | |
| Particle Size of Carrier (µm) | Median Particle Size of 53 to 38 | | | | |
| Cationic Polymer | PAS-M-1L | PAS-411 | PAS-92 | | - |
| Class of Amine | Tertiary | Secondary | Secondary | | - |
| Weight Average Molecular Weight | 5, 000 | 40, 000 | 5, 000 | | - |
| Unit Molecular Weight with respect to Amine | 147. 65 | 172. 31 | 197. 68 | | - |
| pH of Polymer | 9 | 7 | 9 | 7 | - |
| Polymer Concentration (wt%) | 10 | 10 | 10 | 2. 5 | - |
| Bonding Method | Electrostatic Interaction | | | | - |
| Crosslinking Agent | 1,3-Dichloropropane | Epichlorohydrin | | | - |
| Crosslinking Agent Concentration (vol%) | 3 | 3 | 3 | 12. 5 | - |
| Crosslinking Agent/Polymer (molar ratio) | 0. 467 | 0. 659 | 0. 756 | 12. 606 | - |
| Silane Coupling Agent | - | - | - | - | - |
| Blood Cell Separation Ability | Δ | ○ | ○ | ○ | × |
| Desorption of Polymer through pH Change | not desorbed | not desorbed | not desorbed | not desorbed | - |

**[Table 9]**

| | Example | |
|---|---|---|
| | 14 | 15 |
| Carrier | Silica Beads | |
| Particle Size of Carrier (µm) | 1. 5 | |
| Cationic Polymer | PAS-92 | |
| Class of Amine | Secondary | |
| Weight Average Molecular Weight | 5, 000 | |
| Unit Molecular Weight with respect to Amine | 197. 68 | |
| pH of Polymer | 7 | |
| Polymer Concentration (wt%) | 10 | |
| Bonding Method | Silane Coupling | |
| Crosslinking Agent | Epichlorohydrin | |
| Crosslinking Agent Concentration (vol%) | 3 | |
| Crosslinking Agent/Polymer (molar ratio) | 0. 756 | |
| Silane Coupling Agent | Acrylic Silane | Epoxy Silane |
| Blood Cell Separation Ability | ○ | ○ |
| Desorption of Polymer through pH Change | not desorbed | not desorbed |

As is understood from the above-described results, the blood cell separation agents of Examples 1 to 15 all had excellent blood cell separation ability, and the polymers were not desorbed even under acidic conditions. On the contrary, the blood cell separation agent of Comparative Example 2, which was prepared without a crosslinking process, was found to be liable to exhibit lowered blood cell separation ability (as compared with those of Examples 1 and 2), and the polymer was desorbed under acidic conditions. Accordingly, it was revealed that when Specific Polymer(s) are intermolecularly and/or intramolecularly crosslinked, the blood cell separation ability is improved, and the polymer is not desorbed from the inorganic carrier even through pH change.

From the results from evaluation of the blood cell separation agents of Examples 8 and 9, the weight average molecular weight was found noteworthy. The blood cell separation ability tends to be increased as the weight average molecular weight is increased. Assuming that the number of polymers that can be bonded to a carrier is constant, it is presumed that the blood cell separation ability would be improved as the weight average molecular weights of the polymers are higher because the number of functional groups available for blood cell capture is increased.

## Claims

1. An agent for use in blood cell separation comprising an inorganic carrier, and polymer(s) having a constituent unit with amino group(s),
wherein at least some of the amino group(s) are a primary amino group, a secondary amino group, or a tertiary amino group,
at least some of the polymer(s) are carried on the inorganic carrier, and
others of the polymer(s) are linked to the polymer(s) carried on the inorganic carrier by crosslinking.

2. The agent for use in blood cell separation according to claim 1, wherein at least some of the amino group(s) are a secondary amino group, or a tertiary amino group.

3. The agent for use in blood cell separation according to claim 1 or 2, wherein the crosslinking is made by a crosslinking agent that is a compound having, in total, at least two functional groups capable of forming a covalent bond with the amino group(s).

4. The agent for use in blood cell separation according to any one of claims 1 to 3, wherein the polymer(s) are (a1) an allylamine (co)polymer, (a2) a diallylamine (co)polymer, or (a3) a polyethyleneimine.

5. The agent for use in blood cell separation according to claim 4, wherein the polymer(s) are (a2) a diallylamine (co)polymer or (a3) a polyethyleneimine.

6. The agent for use in blood cell separation according to any one of claims 1 to 5, wherein the weight average molecular weight of the polymer is in the range of 3,000 to 200,000.

7. The agent for use in blood cell separation according to any one of claims 1 to 6, wherein the inorganic carrier is an inorganic particle having a primary particle size between 0.05 and 500 µm.

8. The agent for use in blood cell separation according to any one of claims 1 to 7, wherein the polymer(s) is carried on the inorganic carrier by adsorption through electrostatic interaction.

9. The agent for use in blood cell separation according to any one of claims 1 to 7, wherein the polymer(s) are carried on the inorganic carrier by a covalent bond.

10. The agent for use in blood cell separation according to claim 9, wherein the covalent bond is formed by silane coupling.

11. A method of separating blood cell, comprising the step of contacting a specimen containing blood cells with the agent for use in blood cell separation according to any one of claims 1 to 10.

12. A method for producing an agent for use in blood cell separation, comprising the steps of:
carrying polymer(s) having a constituent unit with amino group(s) on an inorganic carrier; and
reacting, before or after carrying the polymer(s) on the inorganic carrier, the polymer(s) with a crosslinking agent to link the polymer(s) with each other by crosslinking,
wherein at least some of the amino group(s) are a primary amino group, a secondary amino group, or a tertiary amino group.

13. The method according to claim 12, wherein at least some of the amino group(s) are a secondary amino group, or a tertiary amino group.

14. The method according to claim 12 or 13, wherein the polymer(s) are (a1) an allylamine (co)polymer, (a2) a diallylamine (co)polymer, or (a3) a polyethyleneimine.

15. The method according to claim 14, wherein the polymer(s) are (a2) a diallylamine (co)polymer, or (a3) a polyethyleneimine.

16. The method according to any one of claims 12 to 15, wherein the polymer(s) are carried on the inorganic carrier by adsorption through electrostatic interaction and/or by a covalent bond.

17. The method according to claim 16, wherein the polymer(s) are covalently bonded to the inorganic carrier with a silane coupling agent.

18. The method according to claim 16, wherein the polymer(s) are contacted with the inorganic carrier in an alkaline solution to be adsorbed on the inorganic carrier by electrostatic interaction.

19. The method according to any one of claims 12 to 18, wherein the crosslinking agent is a compound having, in total, at least two functional groups capable of forming a covalent bond with the amino group(s).
